# EUROPÄISCHE PATENTSCHRIFT

(11) **EP 1 556 391 B1**
(45) Veröffentlichungstag und Bekanntmachung des Hinweises auf die Patenterteilung: **01.08.2007**
(21) Anmeldenummer: 03750666.4
(22) Anmeldetag: 01.10.2003
(51) Int. Cl.: C07F 9/09, C07F 9/10

(54) **LIPIDANALOGE PHOSPHORS URETRIESTER**
LIPID-ANALOG PHOSPHORIC ACID TRIESTER
TRIESTERS D'ACIDE PHOSPHORIQUE ANALOGUES DE LIPIDES

(30) Priorität: 01.10.2002 DE 10245909
(43) Veröffentlichungstag der Anmeldung: 27.07.2005
(73) Patentinhaber: Max-Planck-Gesellschaft zur Förderung der Wissenschaften e.V., 80539 München (DE)
(72) Erfinder: EIBL, Hansjörg, 37120 Bovenden (DE)
(74) Vertreter: Dey, Michael
(86) Internationale Anmeldenummer: PCT/EP2003/010870
(87) Internationale Veröffentlichungsnummer: WO 2004/031199

(56) Entgegenhaltungen:
- WO-A-95/01163
- DE-A- 19 622 224
- US-A- 5 651 981
- US-A- 5 855 910
- US-A- 5 891 714
- DATABASE CA [Online] CHEMICAL ABSTRACTS SERVICE, COLUMBUS, OHIO, US; CREMLYN, R. J. W. ET AL: "Studies of organophosphorochloridates. Reactions of cholesteryl phosphorochloridates with alcohols" XP002265021 gefunden im STN Database accession no. 86:121620 & PHOSPHORUS AND THE RELATED GROUP V ELEMENTS (1976), 6(3-4), 201-5, 1976,
- DATABASE CROSSFIRE BEILSTEIN [Online] Beilstein Institut zur Förderung der Chemischen Wissenschaften, Frankfurt am Main, DE; 8. März 2000 (2000-03-08), XP002277994 Database accession no. BRN 8384216
- DATABASE CROSSFIRE BEILSTEIN [Online] Beilstein Institut zur Förderung der Chemischen Wissenschaften, Frankfurt am Main, DE; XP002277995 Database accession no. BRN 6788943 & SHVETS ET AL.: J. ORG. CHEM. USSR (ENGL. ÜBERSETZ.), Bd. 5, 1969, Seiten 1978-1983,
- DATABASE CROSSFIRE BEILSTEIN [Online] Beilstein Institut zur Förderung der Chemischen Wissenschaften, Frankfurt am Main, DE; XP002277996 Database accession no. BRN 7400057 & HARVEY, D. J.: J. MASS. SPECTROM., Bd. 30, Nr. 9, 1995, Seiten 1333-1346,
- MURAKAMI, K. ET AL.: "An Efficient Synthesis of Unsymmetrical Optically Active Phosphatidyl Glycerol" J. ORG. CHEM., Bd. 64, 1999, Seiten 648-651, XP002277991
- KEANA ET AL.: "A Short, Flexible Route to Symmetrically and Unsymmetrically Substituted Diphosphatidylglycerols (Cardiolipins)" J. ORG. CHEM., Bd. 51, Nr. 12, 1986, Seiten 2297-2299, XP002277992
- BRUZIK, K. ET AL.: "General Method for the Synthesis of Glycerophospholipids" J. ORG. CHEM., Bd. 51, Nr. 12, 1986, Seiten 2368-2370, XP002277993
- MACDONALD R C ET AL: "O-ETHYLPHOSPHATIDYLCHOLINE: A METABOLIZABLE CATIONIC PHOSPHOLIPID WHICH IS A SERUM-COMPATIBLE DNA TRANSFECTION AGENT" JOURNAL OF PHARMACEUTICAL SCIENCES, AMERICAN PHARMACEUTICAL ASSOCIATION. WASHINGTON, US, Bd. 88, Nr. 9, September 1999 (1999-09), Seiten 896-904, XP000851255 ISSN: 0022-3549
- DATABASE CROSSFIRE BEILSTEIN [Online] Beilstein Institut zur Förderung der Chemischen Wissenschaften, Frankfurt am Main, DE; XP002277997 Database accession no. BRN 8468920 & KIM ET AL.: PHARM. RES., Bd. 18, Nr. 1, 2001, Seiten 54-60,

## Beschreibung

Die vorliegende Erfindung betrifft neue Phosphorsäuretriester, welche apolare Lipidstrukturen enthalten.

US 5,855,910 sowie US 5,891,714 beschreiben Phosphorsäuretriester-Verbindungen, welche einen Diacylglycerin-, einen Cholin- und einen Alkylrest enthalten. Die beschriebenen Verbindungen weisen jedoch keine freien Hydroxylgruppen auf. J. Keana et al., J. Org. Chem. 1986, 51, 2297-2299, beschreibt als Zwischenverbindung zur Herstellung von Kardiolipinen einen Phosphorsäuretriester enthaltend einen Dipalmitoylglycerin-, einen Glycerin- sowie einen Methylrest. Diese Zwischenverbindung wird dann weiter mit Diazomethan umgesetzt.

Die erfindungsgemäßen Triester können insbesondere als Liposomenbestandteile Verwendung finden.

Ein Gegenstand der Erfindung ist eine Verbindung der Formel (I) 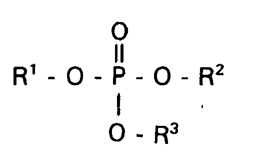 worin R¹ einen Rest darstellt, ausgewählt aus Cholesterin, Diacylglycerinen, Dialkylglycerinen, Acylalkylglycerinen, Ceramiden, primären oder sekundären Alkoholen mit 12 bis 24 C-Atomen oder Acylglycero-benzylethern, R² einen Rest darstellt, ausgewählt aus Ethanolamin, N-Methylethanolamin, Propanolamin, Cholin, Glycerin, Oligoglycerinen, Glycoglycerinen oder Serin, welche gegebenenfalls Schutzgruppen enthalten können und R³ einen Rest darstellt, der eine der für R² angegebenen Bedeutungen hat.

Bei den erfindungsgemäßen Verbindungen handelt es sich um Lipid-analoge Phosphorsäuretriester, insbesondere um Phosphorsäuretriester mit mehrfachen Hydroxylgruppen pro Phosphoratom, insbesondere mindestens zwei Hydroxylgruppen pro Phosphoratom, mehr bevorzugt mindestens drei Hydroxylgruppen pro Phosphoratom und noch mehr bevorzugt mindestens vier Hydroxylgruppen pro Phosphoratom.

Die erfindungsgemäßen Phosphorsäuretriester enthalten einen Rest R', welcher eine apolare Lipidstruktur enthält. Geeignete Strukturen für R¹ sind insbesondere Cholesterinreste, sodass es sich bei bevorzugten Phosphoräuretriestern um Cholesteryl-Verbindungen handelt.

Ein weiterer bevorzugter Rest für R¹ ist Diacylglycerin, wobei die Acylgruppen jeweils unabhängig vorzugsweise 12 bis 28, insbesondere 13 bis 27 und mehr bevorzugt 14 bis 26 Kohlenstoffatome enthalten. Bei den Acylresten kann es sich um gesättigte oder ein- oder mehrfach, insbesondere zwei- oder dreifach ungesättigte Reste handeln. Besonders bevorzugt sind die Acylglycerine, welche ungesättigte Fettsäurereste, wie beispielsweise Reste von Ölsäure, Linolsäure oder Linolensäure enthalten.

Bei dem Rest R¹ kann es sich erfindungsgemäß weiterhin um einen Dialkylglycerinrest handeln, wobei die Alkylreste darin jeweils unabhängig voneinander vorzugsweise 1 bis 28, insbesondere 12 bis 26, noch mehr bevorzugt 14 bis 24 Kohlenstoffatome aufweisen. Die Alkylreste im Dialkylglycerinrest können gesättigt oder ein- oder mehrfach, insbesondere zwei- oder dreifach ungesättigt sein. Bevorzugt sind die Reste (Z)-9-Octadecenyl-, (Z.Z.)-9.12-Octadecandienyl-, (Z.Z.Z)-9.12.15-Octadecantrienyl sowie lipophile Grundkörper mit pharmazeutischer Wirkung, wie 1-Octadecyl-2-methyl-sn-glycerin.

Weiterhin kann R¹ für einen Ceramidrest stehen. Ceramide sind körpereigene, besonders in der Hirnsubstanz und im Myelin des ZNS gebunden anzutreffende lipophile Amide der allgemeinen Formel (IV) 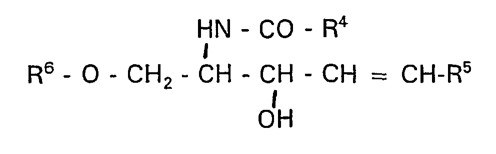 wobei R⁴ ein langkettiger Fettsäurerest, insbesondere ein Fettsäurerest mit 12 bis 28 C-Atomen, R⁵ ein langkettiger Alkylrest, insbesondere ein Alkylrest mit 12 bis 28 C-Atomen und R⁶ = H ist.

R¹ kann weiterhin für einen Rest eines primären oder sekundären Alkohols mit 12 bis 24 Kohlenstoffatomen, insbesondere 13 bis 22 Kohlenstoffatomen stehen, wobei die Alkohole gesättigt oder ein- oder mehrfach ungesättigt sein können.

R¹ kann weiterhin für einen Acylglycero-benzylether-Rest stehen, wobei solche Verbindungen insbesondere als Ausgangsprodukte für die Synthese von Lysophospholipiden eingsetzt werden können.

In den erfindungsgemäßen Verbindungen kann der Rest R¹ in enantiomer reiner Form oder als racemisches Gemisch vorliegen.

R² kann für alle Reste stehen, wie sie in natürlichen Phospholipiden und Sphingomyelinen vorkommen. Insbesondere steht R² für einen Ethanolaminrest, einen N-Methylethanolaminrest oder einen Propanolaminrest, wobei die Reste gegebenenfalls mit geeigneten Schutzgruppen, beispielsweise BOC versehen sind. R² kann weiterhin für einen Cholinrest stehen. Bevorzugt steht R² für -CH₂-CH₂-N⁺ (CH₃)₃. R² kann weiterhin für einen Glycerinrest (-CH₂-CH(OH)-CH₂(OH)) stehen sowie für einen Oligoglycerin-, insbesondere einen Di- oder Triglycerinrest. Weitere geeignete Reste R² sind Glycoglycerine, sowie Serinreste. Auch die Glycerin- und Serinreste können gegebenenfalls mit geeigneten Schutzgruppen versehen sein.

R³ stellt einen Rest dar, der eine der oben für R² angegebenen Bedeutungen hat. Dabei, können biologisch hochaktive Strukturen gebildet werden, die als neue kationische Lipide eine eine große Bedeutung haben. Solche kationischen Lipide können beispielsweise für die Gentransfektion eingesetzt werden.

Die erfindungsgemäßen Verbindungen sind zwischen pH 3 und pH 8 sehr stabil und können insbesondere als Liposomenbestandteile Verwendung finden.

Die Erfindung betrifft weiterhin ein Verfahren zur Herstellung einer Verbindung nach Formel (I), welches dadurch gekennzeichnet ist, dass man eine Verbindung der Formel (II) mit einer Verbindung der Formel (III) HO-R³ verestert.

Die Verbindungen leiten sich von Phospholipiden her und entstehen beispielsweise aus 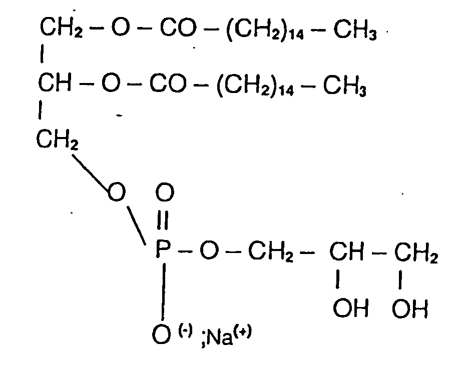 durch Veresterung des Phosphorsäurediesters mit Glycerin:

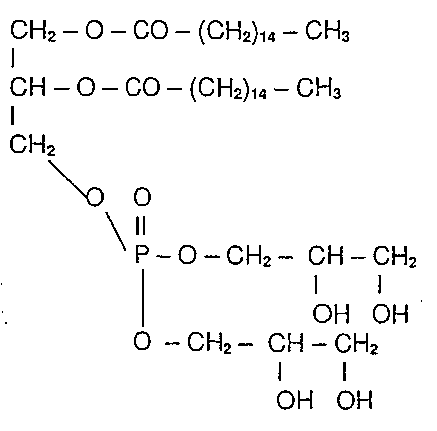

Entsprechend können auch Cholesterinderivate erhalten werden

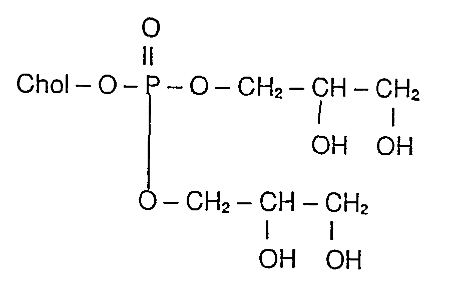

Diese Verbindungen sind in unterschiedlichen Ausführungsformen herstellbar. Sie können auch Oligoglycerine, z.B. anstelle von Glycerin beispielsweise Glyceroglycerin, Diglyceroglycerin oder Triglyceroglycerin enthalten. Schematisch besitzt z.B. Cholesterinphospho-monoglycerintriglyceroglycerin folgende Strukturformel:

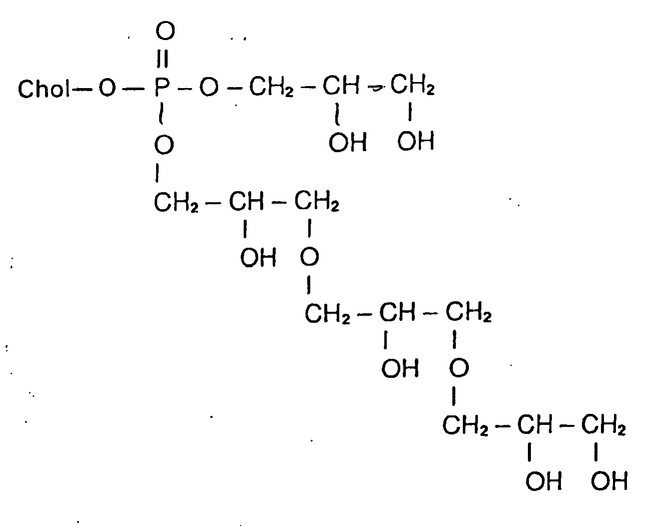

Die erfindungsgemäßen Verbindungen sind insbesondere zur Herstellung von Liposomen bzw. als Liposomenbestandteile geeignet. Sie verleihen Liposomen besondere Eigenschaften, z.B. lange Zirkulationszeiten im Blut, gezielte Anreicherung in der Leber oder auch fast exklusive Aufnahme in der Milz. Mit Hilfe der erfindungsgemäßen Phosphorsäuretriester können auch Liposomen mit neuen Eigenschaften gebildet werden, die eine hohe Serumstabilität aufweisen, lange Zirkulationszeiten besitzen und exklusiv in der Milz angereichert werden. Lange Zirkulationszeiten sind aber auch deshalb besonders wichtig, weil die Strukturen dann nicht, wie bekannte Liposomen, in der Leber angereichert werden sonderen andere Ziele, wie z.B. die Milz treffen oder besonders wichtig, durch Tumorzellen aufgenommen werden können. Die erfindungsgemäßen Verbindungen können deshalb auch zur Behandlung von Krebserkrankungen eingesetzt werden.

Die Erfindung betrifft weiterhin einen neuen Syntheseweg unter Verwendung der erfindungsgemäßen Phosphorsäuretriester als Zwischenstufe. Ein besonderer Vorteil des erfindungsgemäßen Synthesewegs ist, dass die in früheren Synthesen benutzte Reaktionsführung (a) vermieden wird und nach (b) die wichtige Verbindung 1.2-Dioleyl-sn-glycero-3-phosphoglycerin oder entsprechende Verbindungen unter neutralen Bedingungen freigelegt werden:
R₁ = 1.2 - Dioleoyl - sn - glycerin

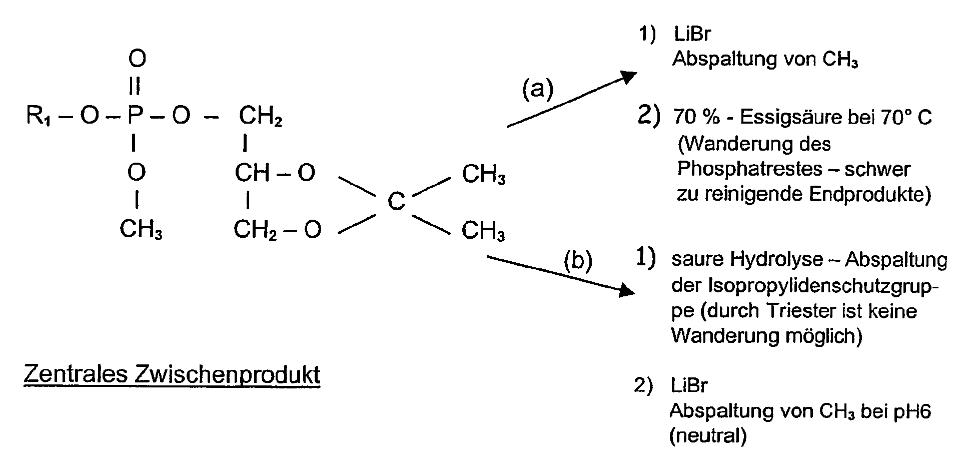

Ein wesentlicher Vorteil bei der neuen Syntheseführung ist die Möglichkeit, apolare Zwischenprodukte möglichst weit in der Synthese voranzubringen, sodass polare Strukturen erst am Ende des Verfahrens eingeführt werden. Dies wird im Folgenden an einem Beispiel erläutert. Cardiolipine und analoge Verbindungen sind komplizierte Strukturen, die synthetisch in kg-Mengen nur äußerst schwer zugänglich sind. Mit Hilfe unserer neuen Synthesestrategie ist dies aber gut möglich.

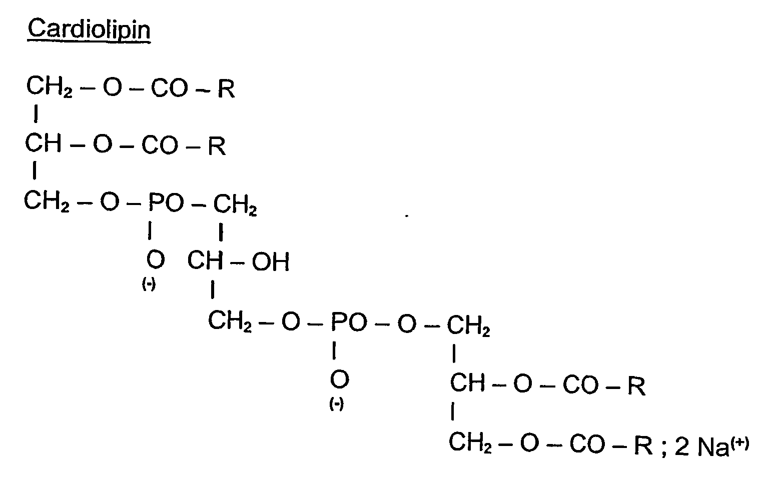

Die Synthese wird für das Beispiel R = Palmitinsäure beschrieben. Ausgangsprodukt ist 1.2-Dipalmitoyl-sn-glycerin, das mit Phosphoroxychlorid in THF mit Triethylamin als Base in gewohnter Weise zu 1.2-Dipalmitoyl-sn-glycero-3-phosphorsäuredichlorid umgewandeltwird:

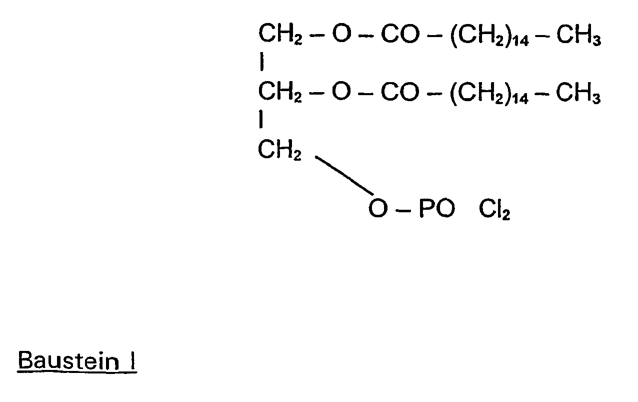

Der einfache Weg zur Synthese von Cardiolipin, direkte Umsetzung mit 2-Benzyl-glycerin führt leider zur überwiegenden Bildung des entsprechenden Phospholans und ist nicht praktikabel:

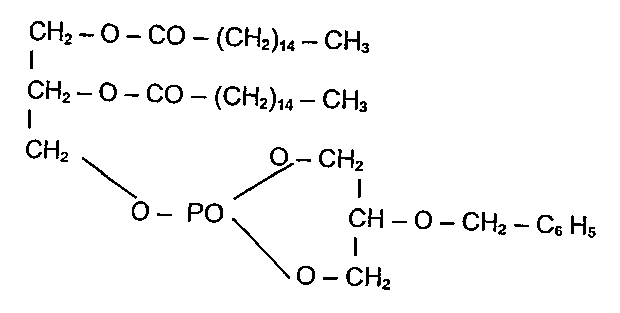

Deshalb muss Baustein II verwendet werden, ein geschütztes Glycerinderivat:

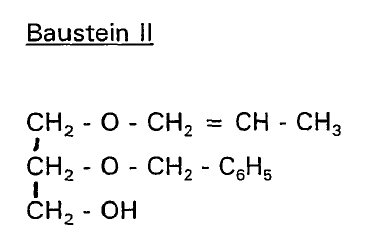

Die Verknüpfung von Baustein I mit Baustein II in THF mit Triethylamin führt dann zu Baustein III:

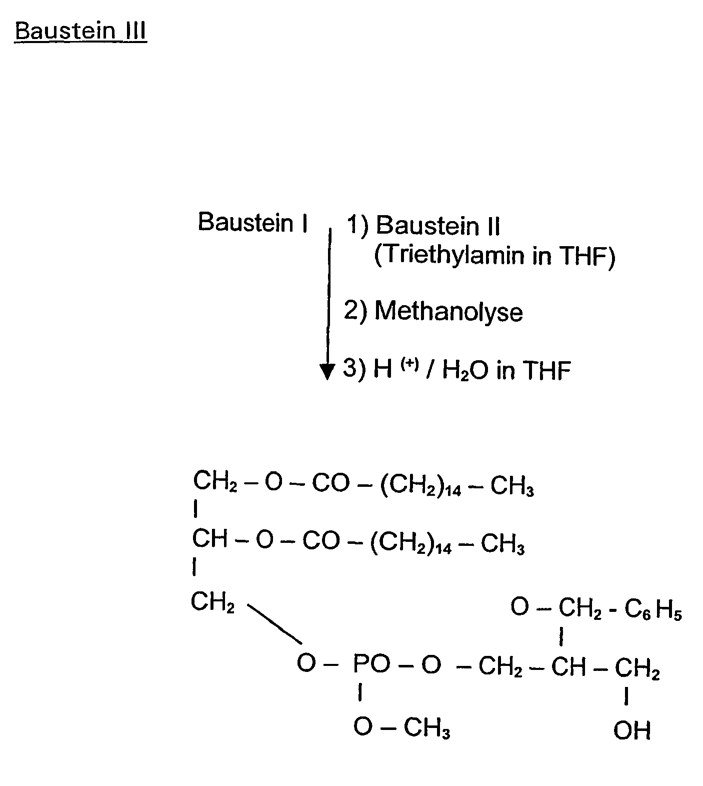

Baustein III kann dann mit Baustein I in üblicher Weise umgesetzt werden zu dem direkten Vorprodukt von Cardiolipin, das dann durch

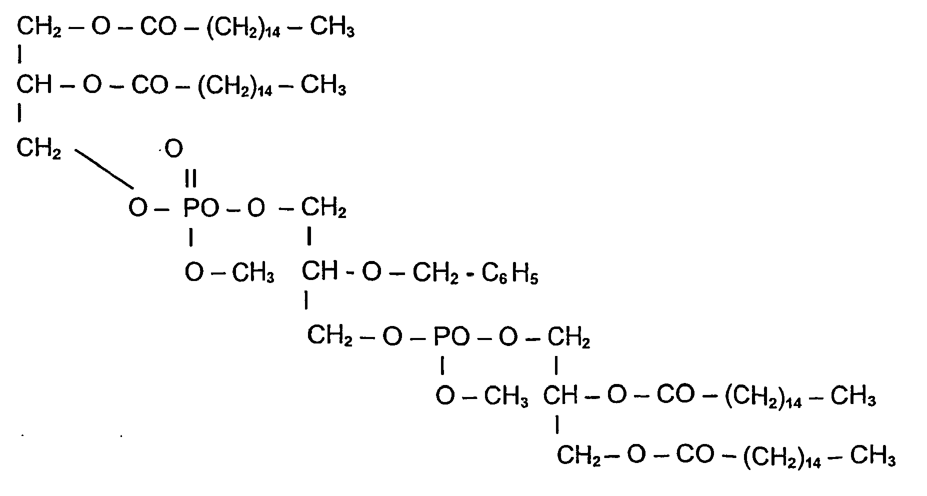

Methanolyse in dem Dimethylester überführt wird. Durch katalytisch Hydrogenolyse wird dann die Hydroxylgruppe am mittleren Glycerin freigelegt. Die Methylgruppen werden durch LiBr bei neutralem pH entfernt - das Endprodukt ist Cardiolipin.

Die Beschreibung wird durch die folgenden Beispiele weiter erläutert.

### Beispiel 1

1) Cholesteryl - phospho - diglycerin
   C₃₃ H₅₉ O₈ P (MG 614.801)
2) Cholesteryl - phospho - glycerin - glyceroglycerin
   C₃₆ H₆₅ O₁₀ P (MG 688.880)
3) Cholesteryl - phospho - di - glykoglycerin
   C₃₇H₆₇ O₁₀ P (MG 702.907)
4) 1.2 - Dimyristoyl - sn - glycero - 3 - phospho - diglycerin
   C₃₇ H₇₃ O₁₂ P (MG 740.953)
5) 1.2 - Dipalmitoyl - sn - glycero - 3 - phospho - diglycerin
   C₄₁ H₈₁ O₁₂ P (MG 797.061)
6) 1.2 - Distearoyl - sn - glycero - 3 - phospho - diglycerin
   C₄₅ H₈₉ O₁₂ P (MG 853.169)
7) 1.2 - Dioleoyl - sn - glycero - 3 - phospho - diglycerin
   C₄₅H₈₅ O₁₂ P (MG 849.137)
8) 1.2 - Dioleoyl - sn - glycero - 3 - phospho - di - glykoglycerin
   C₄₉ H₉₃ O₁₄ P (MG 937.243)
9) 1.2 - Dioleoyl - sn - glycero - 3 - phospho - di - glyceroglycerin
   C₅₁ H₉₇ O₁₆ P (MG 997.295)

### Beispiel 2

1) R₁: 1.2. - Dimyristoyl - glycerin
   R₂: Cholin
   R₃: Glycerin 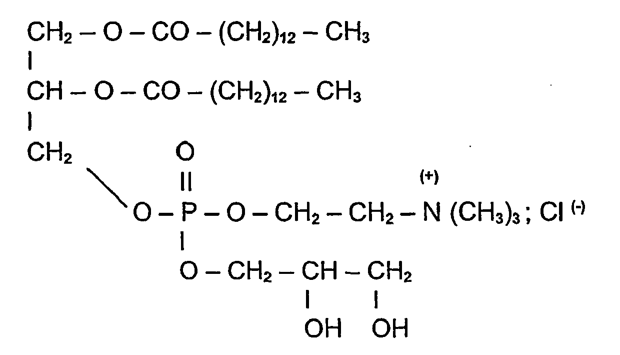 und entsprechende Strukturen mit 1.2. Dioleoylglycerin.
2) R₁: 1.2-Dioleoylglycerin
   R₂: Glycerin
   R₃: Methyl

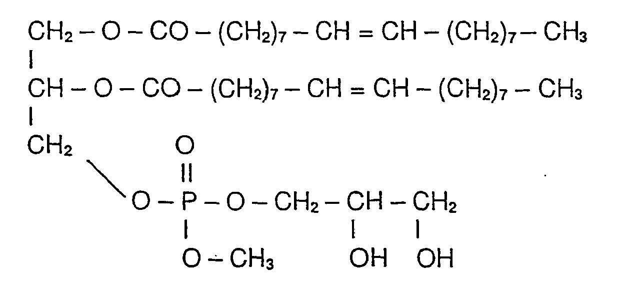

### Beispiel 3

### Liposomen der Zusammensetzung

| | |
|---|---|
| | Molares Verhältnis |
| 1.2 - Distearoyl - sn - glycero - 3 - phosphocholin | 40 % |
| Cholesterin | 30 % |
| Cholesterin - phospho - diglycerin | 20 % |
| Cholesterin - phospho - glycerin, Na⁽⁺⁾-Salz | 10 % |
| | 100% |

reichern sich vorwiegend in der Milz a, während Liposomen der üblichen Zusammensetzung z.B.

| | |
|---|---|
| 1.2 - Distearoyl - sn - glycero - 3 - phosphocholin | 50 % |
| Cholesterin | 40 % |
| 1.2 - Distearoyl - sn - glycero - 3 - phosphoglycerin, | 10 % |
| Na⁽⁺⁾-Salz | 100 % |

hauptsächlich in der Leber angereichert werden.

## Patentansprüche

1. Verbindung der Formel (I) 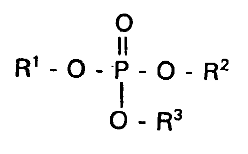 worin R¹ einen Rest darstellt, ausgewählt aus Cholesterin, Diacylglycerinen, Dialkylglycerinen, Acylalkylglycerinen, Ceramiden, primären oder sekundären Alkoholen mit 12 bis 24 C-Atomen oder Acylglycero-benzylethern,
R² und R³ jeweils unabhängig einen Rest darstellt, ausgewählt aus Ethanolamin, N-Methylethanolamin, Propanolamin, Cholin, Glycerin, Oligoglycerinen, Glycoglycerinen oder Serin, welche gegebenenfalls Schutzgruppen enthalten können,
wobei die Verbindung mindestens zwei Hydroxylgruppen pro Phosphoratom aufweist.

2. Verbindung nach Anspruch 1,
**dadurch gekennzeichnet,**
**dass** R¹ für einen Cholesterinrest steht.

3. Verbindung nach Anspruch 1,
**dadurch gekennzeichnet,**
**dass** R¹ für einen 1-Octadecyl-2-methyl-sn-glycerin-Rest steht.

4. Verbindung nach einem der vorhergehenden Ansprüche,
**dadurch gekennzeichnet,**
**dass** R² für einen Glycerinrest steht.

5. Verbindung nach einem der vorhergehenden Ansprüche,
**dadurch gekennzeichnet,**
**dass** R³ für einen Glycerinrest steht.

6. Verbindung nach einem der vorhergehenden Ansprüche, ausgewählt aus Cholesteryl-phospho-diglycerin, Cholesteryl-phospho-glyceringlyceroglycerin, Cholesteryl-phospho-di-glycoglycerin, 1,2-Dimyristoyl-sn-glycero-3-phospho-diglycerin, 1,2-Dipalmitoyl-sn-glycero-3-phospho-diglycerin, 1,2-Distearoyl-sn-glycero-3-phospho-diglycerin, 1,2-Dioleoyl-sn-glycero-3-phospho-diglycerin, 1,2-Dioleoyl-sn-glycero-3-phospho-di-glycoglycerin, 1,2-Dioleoyl-sn-glycero-3-phospho-di-glyceroglycerin.

7. Verfahren zur Herstellung einer Verbindung nach Formel (I) gemäß einem der Ansprüche 1 bis 6,
**dadurch gekennzeichnet,**
**dass** man eine Verbindung der Formel (II) 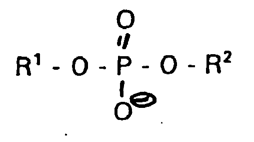 mit einer Verbindung der Formel (III)
HO-R³
verestert.

8. Liposom, enthaltend eine Verbindung nach einem der Ansprüche 1 bis 6.

9. Arzneimittel enthaltend eine Verbindung nach einem der Ansprüche 1 bis 6 oder ein Liposom nach Anspruch 8.

10. Verwendung einer Verbindung nach einem der Ansprüche 1 bis 6 oder eines Liposoms nach Anspruch 8 zur Herstellung eines Arzneimittels zur Behandlung von Krebs.

## Claims

1. Compound of the formula (I) 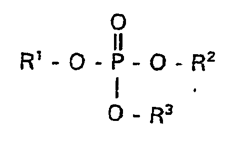 in which R¹ is a residue selected from cholesterol, diacylglycerols, dialkylglycerols, acylalkyl-glycerols, ceramides, primary or secondary alcohols having 12 to 24 C atoms or acylglycerobenzyl ethers, R² and R³ in each case independently is a residue selected from ethanolamine, N-methylethanolamine, propanolamine, choline, glycerol, oligoglycerols, glycoglycerols or serine, each of which may optionally comprise protective groups, where the compound has at least two hydroxyl groups per phosphorus atom.

2. Compound according to claim 1, **characterized in that** R¹ is a cholesterol residue.

3. Compound according to claim 1, **characterized in that** R¹ is a 1-octadecyl-2-methyl-sn-glycerol residue.

4. Compound according to any of the preceding claims, **characterized in that** R² is a glycerol residue.

5. Compound according to any of the preceding claims, **characterized in that** R³ is a glycerol residue.

6. Compound according to any of the preceding claims, selected from cholesteryl-phospho-diglycerol, cholesteryl-phospho-glycerol-glyceroglycerol, cholesteryl-phospho-di-glycoglycerol, 1,2-dimyristoyl-sn-glycero-3-phospho-diglycerol, 1,2-dipalmitoyl-sn-glycero-3-phospho-diglycerol, 1,2-distearoyl-sn-glycero-3-phospho-diglycerol, 1,2-dioleoyl-sn-glycero-3-phospho-diglycerol, 1,2-dioleoyl-sn-glycero-3-phospho-di-glycoglycerol, 1,2-dioleoyl-sn-glycero-3-phospho-di-glyceroglycerol.

7. Process for preparing a compound according to formula (I) according to any of claims 1 to 6, **characterized in that** a compound of the formula (II) 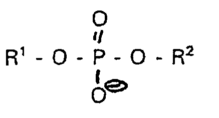 is esterified with a compound of the formula (III) HO-R³.

8. Liposome comprising a compound according to any of claims 1 to 6.

9. Medicament comprising a compound according to any of claims 1 to 6 or a liposome according to claim 8.

10. Use of a compound according to any of claims 1 to 6 or of a liposome according to claim 8 for producing a medicament for the treatment of cancer.

## Revendications

1. Composé de formule (I) 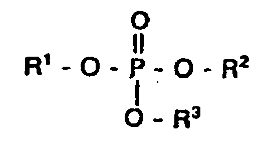 dans laquelle R¹ représente un reste choisi parmi le cholestérol, des diacylglycérols, des dialkylglycérols, des acylalkylglycérols, des céramides, des alcools primaires ou secondaires de 12 à 24 atomes de carbone ou des éthers d'acylglycéro-benzyle,
R² et R³ représentent chacun indépendamment un reste choisi parmi l'éthanolamine, la N-méthyléthanolamine, la propanolamine, la choline, le glycérol, des oligoglycérols, des glycoglycérols ou la sérine, qui peuvent éventuellement contenir des groupes protecteurs,
le composé contenant au moins 2 groupes hydroxyle par atome de phosphore.

2. Composé selon la revendication 1, **caractérisé en ce que** R¹ représente un reste de cholestérol.

3. Composé selon la revendication 1, **caractérisé en ce que** R¹ représente un reste de 1-octadécyl-2-méthyl-sn-glycérol.

4. Composé selon l'une des revendications précédentes, **caractérisé en ce que** R² représente un reste de glycérol.

5. Composé selon l'une des revendications précédentes, **caractérisé en ce que** R³ représente un reste de glycérol.

6. Composé selon l'une des revendications précédentes, choisi parmi le cholestérylphosphodiglycérol, le cholestérylphosphoglycérol-glycéroglycérol, le cholestérylphosphodiglycoglycérol, le 1,2-dimyristoyl-sn-glycéro-3-phosphodiglycérol, le 1,2-dipalmitoyl-sn-glycéro-3-phosphodiglycérol, le 1,2-distéaroyl-sn-glycéro-3-phosphodiglycérol, le 1,2-dioléoyl-sn-glycéro-3-phosphodiglycérol, le 1,2-dioléoyl-sn-glycéro-3-phosphodiglycoglycérol, le 1,2-dioléoyl-sn-glycéro-3-phosphodiglycéroglycérol.

7. Procédé de préparation d'un composé de formule (I) selon l'une des revendications 1 à 6, **caractérisé en ce que** l'on estérifie un composé de formule (II) 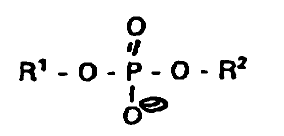 avec un composé de formule (III)
HO-R³.

8. Liposome contenant un composé selon l'une des revendications 1 à 6.

9. Médicament contenant un composé selon l'une des revendications 1 à 6 ou un liposome selon la revendication 8.

10. Utilisation d'un composé selon l'une des revendications 1 à 6 ou d'un liposome selon la revendication 8 pour la préparation d'un médicament destiné au traitement du cancer.
